# EUROPEAN PATENT SPECIFICATION

(11) **EP 1 726 282 B1**
(45) Date of publication and mention of the grant of the patent: **02.11.2011**
(21) Application number: 06010285.2
(22) Date of filing: 18.05.2006
(51) Int. Cl.: A61H 23/02

(54) **Massage vibrator apparatus**
Vibrationsmassagevorrichtung
Vibromasseur

(30) Priority: 20.05.2005 IT TO20050346
(43) Date of publication of application: 29.11.2006
(73) Proprietor: Becchis, Luigi, 10022 Carmagnola TO (IT)
(72) Inventor: Becchis, Luigi, 10022 Carmagnola TO (IT)
(74) Representative: Aprà, Mario

(56) References cited:
- WO-A-96/32915
- US-A- 4 732 140
- US-A- 5 611 771
- US-A- 5 764 776
- US-A- 6 022 328
- US-A1- 2005 059 911

## Description

The present invention relates to a massage vibrator apparatus for the execution of massages with therapeutic and aesthetic, rehabilitation, sport training purposes.

Massage vibrator apparatuses are known. They generally comprise an electric motor with eccentric means, so-called motor-vibrator, which imparts a vibratory motion to at least one tool, gripped by an operator, who uses it to perform massages on a person's body. Generally, the motor-vibrator is separate from the vibrating tool gripped by the operator. Therefore, the structure of said known massage vibrator apparatuses is relatively complex and bulky and it requires the presence of an operator who, gripping the vibrating tool, performs the massage on the person to be treated.

Said person cannot directly control the massage vibrator apparatus.

Furthermore, various apparatuses have been proposed for massaging applications and comprising vibrating means specific for some parts of the human body (US-A 4,732,140, US-A 6,022,328). Documents U5-A 5,611,771, US 2005/0059911 Al, WO 96/32915 A, US-A-5, 764, 776 exemplify types of known message vibrator apparatuses in which the vibrating means are designed to fit a given part of the human body. These apparatuses are activated by the users through a remote control.

Document US-A 4,732,140 discloses a massage vibrator apparatus which comprises fastening means housing a vibrating motor to be positioned directly on the part to be kneaded. The fastening means comprises belts or strips of various dimensions, for adapting to particular parts of the human body. These strips are fixed to the body by Velcro tissue means in order to adjust their length to the size of the body part to be kneaded. However, it's not possible to adapt a torso belt to a smaller parts of the body, e.g. feet or limb. Thus, a kit of belts or strips of various dimensions is needed for knead the various parts of the human body. Said belts are composed by natural fibres, in particular cotton, interwoven in a manner similar to the weave of a seat belt. To transfer the vibrating motion from the vibrating motor, through the belt, to the concerned body part, the belt is to be fastened strongly around the body part itself, in order to avoid any rubbing between the body skin and the belt and/or vibrating means causing annoying irritations. However a strong fastening of the belt could cause some pain for the user.

All the above-mentioned massage vibrator apparatuses show a structure quite complex and very often are not versatile because they are designed to fit with specific part of the human body.

Moreover, the above-mentioned known massage vibrator apparatuses are firmly and painfully fastened around the user's body by fastening means such as strips and belts, housing vibrating means near to the user's body. If the fastening is not firm, a rubbing action could arise between the body skin and the belt or strip and/or the vibrating means, causing irritation and annoyance to the user.

Furthermore, a fast gripping of the massage vibrator apparatus around the user's body by means of strips or belts firmly fastened does not enable the massage vibrator apparatus to perform massages by a vibrating and oscillating motions, similar to those applied manually.

An object of the present invention is to provide a massage vibrator apparatus which, during the massage treatment, does not require the intervention of any operator.

Another object of the invention is to provide a massage vibrator apparatus, whose operation can be controlled directly by the person who receives the massage. Yet another object of the invention is to provide a massage vibrator apparatus, which enables to perform massages by means of vibrating and oscillating motion, similar to those applied manually, simultaneously acting also in multiple zones of the person' s body.

A further object of the invention is to provide a massage vibrator apparatus, which has a simplified structure and has safe and reliable operation.

In view of these objects, the present invention provides a massage vibrator apparatus, whose essential characteristic is set out in claim 1.

Additional advantageous characteristics are described in the dependent claims.

The aforesaid claims are understood to be wholly incorporated herein.

The present invention shall be more readily apparent from the detailed description that follows, with reference to the accompanying drawings provided purely by way of example, in which:
- Fig. 1 is a perspective, schematic view of a vibration massage unit of the massage vibrator apparatus according to the invention;
- Fig. 2 is a similar view to Fig. 1, in which, however, the vibration massage unit is shown in a different disposition of use;
- Fig. Fig. 3 is also a similar view to Fig. 1, in which, however, the vibration massage unit is shown without a part of a protecting cover of a motor-vibrator;
- Fig. 4 is a perspective, exploded view of the motor-vibrator of the vibration massage unit of Fig. 1;
- Fig. 5 is a perspective, schematic, bottom view of a vibration massage plate of the vibration massage unit of Fig. 1;
- Fig. 6 is a lateral view of the plate of Fig. 5;
- Fig. 7 shows a perspective, schematic view of a mesh framework of said vibration massage plate and of the means for connecting said plate with said motor-vibrator of the vibration massage unit of Fig. 1;
- Fig. 8 is a perspective, schematic, top view of said vibration massage plate of the vibration massage unit of Fig. 1, without said motor-vibrator, and in which said mesh framework and said connection means are also indicated;
- Fig. 9 shows a partially exploded elevation view of a remote control device of the massage vibrator apparatus according to the invention;
- Fig. 10 is a schematic rear perspective view of a control unit of the massage vibrator according to the invention, with respect to which said remote control device is electrically connected;
- Fig. 11 is a schematic front perspective view of said control unit of Fig. 10.

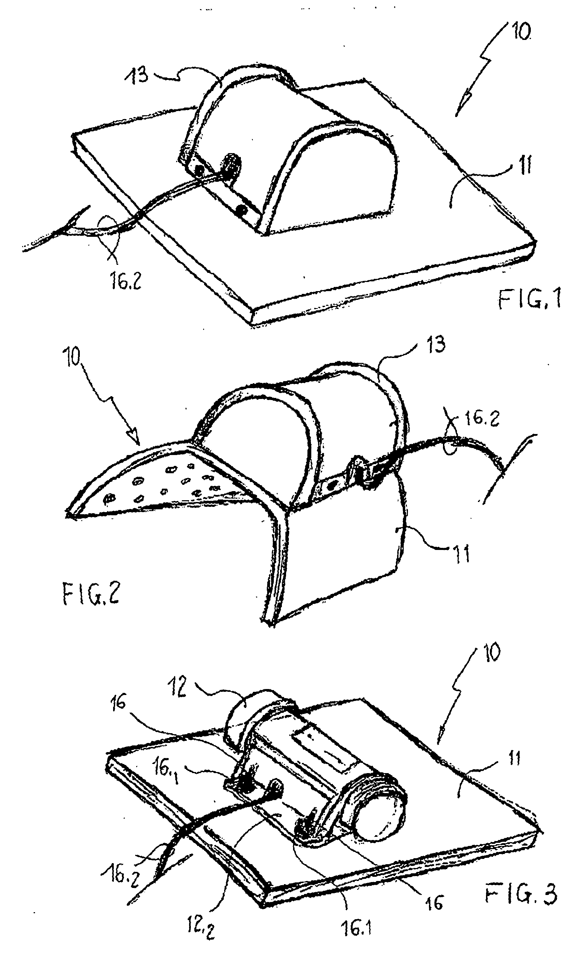

With reference to the drawings, the number 10 (Figures 1 - 3) globally designates a vibration massage unit of the massage vibrator apparatus according to the invention.

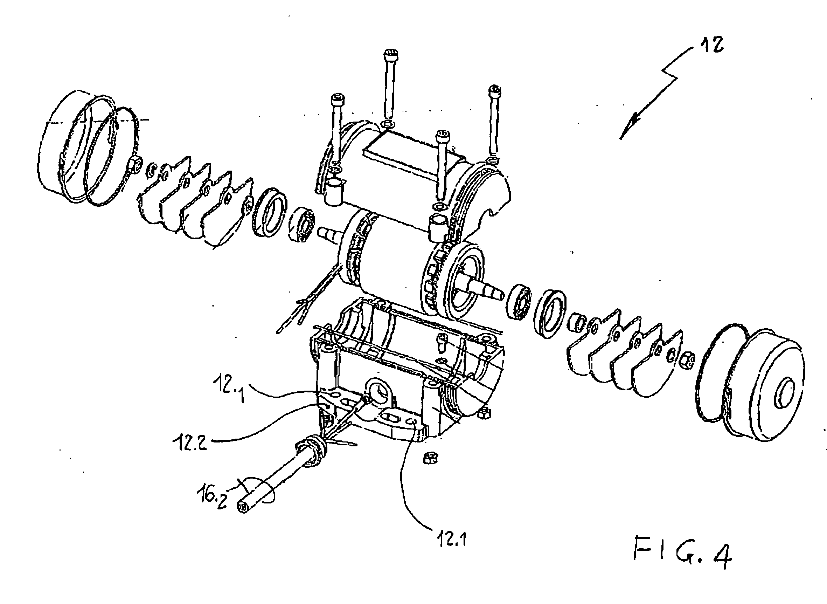

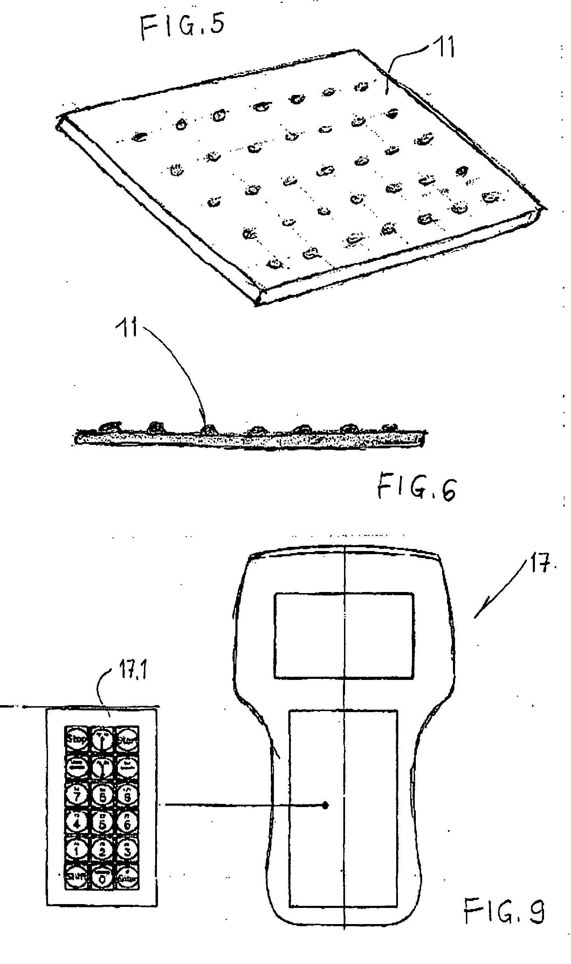

Said vibration massage unit 10 essentially comprises a vibration massage plate 11, quadrangular in shape (Figs. 5 - 8), and a motor-vibrator 12 (Figs. 3, 4), positioned on the upper face and stably connected relative to said plate 11. A lid 13 covers said motor-vibrator 12 connected to the vibration massage plate 11. Said plate 11 is, for example, made of silicone.

As clearly shown in Fig. 4, said motor-vibrator 12 comprises an electric motor, on whose rotating shaft is keyed a plurality of cams (twelve cams in this example), which during the rotation of the shaft (i.e.,when the motor is electrically connected) cause a continuous vibratory, sussultatory and undulatory motion.

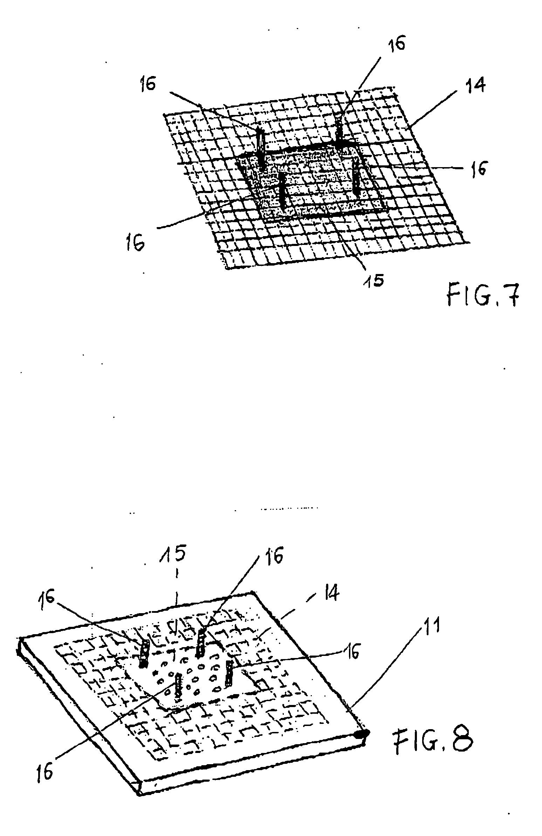

On the other side, in the vibration massage plate 11 is buried a quadrangular metal mesh framework 14 (Figs. 7, 8), which extends substantially throughout the plate. A rectangular rigid metal platelet 15, holed and bearing four fixed screws 16, one for each angle zone, at orthogonal axes to the platelet itself, is applied against said mesh framework 14 and it too is buried in the vibration massage plate 11, at the opposite side from that in which, on said plate 11, is positioned the motor-vibrator 12. Said screws 16 partly extend beyond the upper face of said plate 11 and traverse, with the respective free ends, corresponding through holes 12.1 (Fig. 4), provided in the base 12.2 of the motor-vibrator 12. Corresponding nuts 16.1 fastened by screwing on said screws 16 tightly secure the motor-vibrator 12 to the vibration massage plate 11.

By means of said structure of the vibration massage unit 10, the vibration massage plate 11 can be modelled manually, e.g. as shown in Fig. 2, to adapt it anatomically to the part of the person's body to be massaged, whilst the motor-vibrator 12 remains stably anchored to the plate 11. The lid 13 is substantially semi-cylindrical with projecting edges of its half-bases, to receive and retain a band (not shown) for connecting the vibration massage unit 10 to the part of the person's body to be treated.

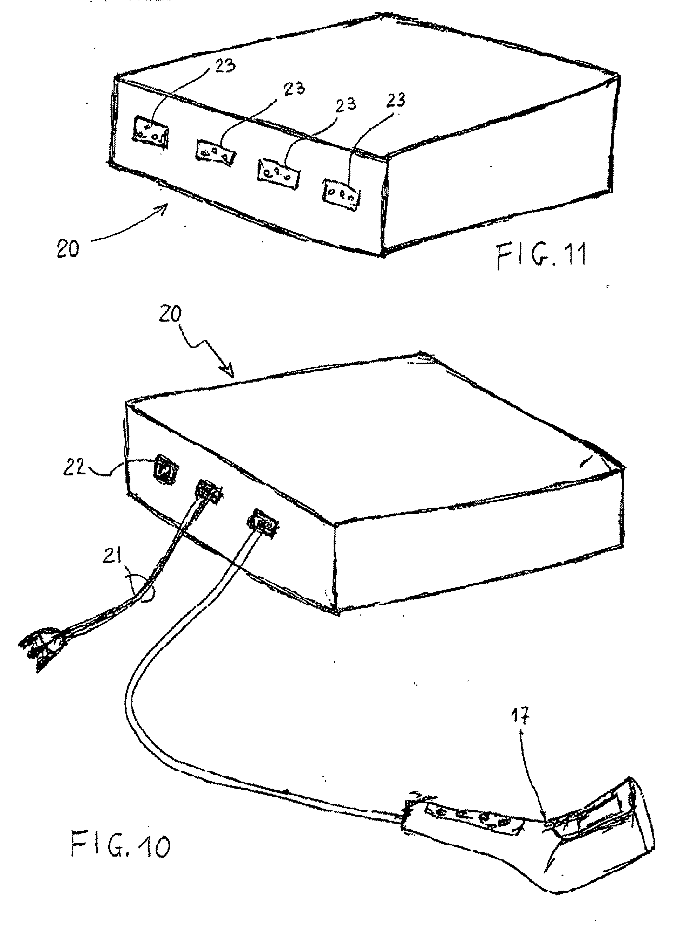

The reference number 20 (Figs. 10, 11) designates a control unit of the massage vibrator apparatus according to the invention. Said control unit 20 is provided, on a lateral face thereof, with an electrical power supply cable 21 for connection to an electrical mains and with an on/off electrical switch 22 to electrically close or open the electrical power supply circuit of the unit.

Moreover, said control unit 20 comprises, on another lateral side thereof, four electrical outlets 23, for the electrical connection of as many vibration massage units 10, each through a respective electrical cable 16.2 branched off the related motor-vibrator 16.

In said control unit 20, the electric circuit means (known in themselves and not shown herein) for connecting each vibration massage unit 10 to the electrical power supply mains (with the switch 22 closed) are remotely controlled by the person receiving the treatment by means of a remote control device 17 (Figs. 9 and 10), electrically connected to said control unit 20.

Said remote control device 17 comprises a push-button panel 17.1, on which the person receiving the treatment enters the number of the vibration massage unit 10 to be controlled (in the illustrated example, from 1 to 4), and which has dedicated push-buttons for controlling respectively the start and the stop of the operation of the unit 10. The rate of rotation of the motor-vibrator 12 of the unit 10 may be varied acting on appropriate push-buttons of said remote control device 17.

The operation of the massage vibrator apparatus according to the invention is readily understandable.

An operator connect the control unit 20 to the electrical mains by means of the power supply cable 21 and electrically connects to the unit the remove control device 17 and at least one (of four) vibration massage units 10, by means of the respective electrical connection outlets of the unit 20. (S)he then applies each vibration massage unit 10 to the selected zone of the body of the person to be treated, manually modelling the respective vibration massage plate 11 to adapt it anatomically to the body. Subsequently, (s)he secures each vibration massage unit on site by means of an elastic band ( not shown) enveloping the lid 13 and the plate 11 in addition to the involved part of the person's body. The person to be treated, having gripped the remote control device 17, controls, independently of the operator, the operation of each vibration massage unit 10 until the end of the treatment, when the operator removes the units and shuts down the apparatus. It will be noted that, during operation, each vibration massage unit 10 is controlled independently of the others. It is also possible, using the remote control device 17 and the control unit 20, to control all the vibration massage units 10 together. Timer means, known in themselves and not shown, may be included in the control unit to predetermine the duration of the treatment.

Each vibration massage unit 10 applies, to the part of the body on which it is positioned, through its anatomically shaped vibration massage plate 11, a vibratory and undulatory massage.

Some of the advantages of the apparatus according to the present invention are:
each vibration massage unit 10 performs a massage similar to a two-handed massage performed manually by an experienced masseur/masseuse;
   - the massage thus performed has considerable therapeutic effectiveness for the person, both on the blood circulation and on the metabolism and on muscle strengthening, as well as great usefulness in improving body tissues with cellulite, with fatty deposits and the like;
   - the anatomical adaptability of the vibration massage plate of each unit 10 enhances the effectiveness of the massage in the cervical, lumbar, dorsal regions of the body, and also when the apparatus is used as a rehabilitation instrument for patients who have undergone surgery.

Moreover, as is readily apparent from the preceding description, the massage vibrator apparatus according to the invention:
- during treatment, does not require any operator's intervention and is directly controlled by the person receiving the massage;
- allows simultaneously to massage multiple zones of the person's body by two, three, four and even more vibration massage units;
- it has a relatively simple structure and its operation is safe and reliable.

Naturally, numerous variants may in practice be provided with respect to what is described and illustrated purely by way of example herein.

Thus, for example, the vibration massage plate can be constructed with other shapes and other materials, e.g. rigid, such as resin.

Moreover, each vibration massage unit may be provided with electrical stimulation means of various types, or with ultrasound plates, or with plates conveying currents or magnetic waves, or with means emitting coloured luminous radiation or infrared radiation, and the like. The remote control device may be connected to the control unit by infrared beams or the like, instead of by electrical cable.

On the other hand, the metal mesh framework buried in the vibration massage plate can be replaced by a flexible, holed metal plate, with holes of various diameters, which substantially has the same modelling characteristics as the mesh framework, but has greater ultimate strength.

## Claims

1. A massage vibrator apparatus for the execution of massages with therapeutic and aesthetic, rehabilitation, sport training purposes, comprising:
- at least one vibration massage unit (10), including a massage vibration plate (11) and a motor-vibrator (12) stably connected relative to said plate (11), and
- control unit (20), provided with electric circuit means for connecting said at least one vibration massage unit (10) to electrical power supply means and remotely controlled by means of a remote control device (17), so that, during treatment, the apparatus does not require the intervention of any operator and is controlled, by means of said remote control device (17), by the person who receives the massage,
**characterised in that** said vibration massage plate (11)is made of silicone and comprises a framework (14) made of metal mesh or a flexible, holed metal plate buried in said vibration massage plate (11), so that it can be manually modelled to adapt it anatomically to the part of the body of the person to be massaged.

2. A massage vibrator apparatus as claimed in claim 1, **characterised in that** said vibration massage plate (11) comprises a little plate (15), bearing fastening means (16) and applied against said framework (14), in such a way as to securely fasten the motor-vibrator (12) to said vibration massage plate (11).

3. A massage vibrator apparatus as claimed in claim 1 and/or 2, **characterised in that** said framework (14) is constituted by a flexible, holed metal plate, with holes of various diameters, which is buried in said vibration massage plate.

4. A massage vibrator apparatus as claimed in claim 2, and/or 3, **characterised in that** said little plate (15) is a rigid, metallic plaque, which bears fastening means (16) and is buried in said vibration massage plate (11), while said fastening means consist of screws (16)which partly project beyond the upper face of said plate (11), traverse, with respective free ends, corresponding through holes (12.1), provided in the base (12.2) of the motor-vibrator (12), and by means of corresponding nuts (16.1) securely fasten the motor-vibrator (12) to said vibration massage plate (11).

## Patentansprüche

1. Vibrationsmassagegerät für die Durchführung von Massagen zum Zwecke therapeutischer und ästhetischer Behandlung, Rehabilitation, Training, umfassend:
- mindestens eine Vibrationsmassageeinheit (10), bestehend aus einer Vibrationsplatte (11) und einem an die genannte Platte (11) fest verbundenen Motorvibrator (12) sowie
- einer Kontrolleinheit (20), ausgerüstet mit Stromkreismitteln für den Anschluss der genannten mindestens einen Vibrationsmassageeinheit (10) an Stromzuführmittel und ferngesteuert mittels eines Fernbedienungsgerätes (17),
sodass das Massagegerät während der Behandlung keinen Eingriff von Seiten eines Bedieners erfordert und mittels der genannten Fernbedienung (17) vom Massage-Empfänger gesteuert wird,
**dadurch gekennzeichnet, dass** genannte Vibrationsplatte (11) aus Silikon ist und ein Gerüst (14) aus Metallnetz oder einer biegsamen Metall-Lochplatte, eingelagert in genannte Vibrationsplatte (11), umfasst, sodass sie manuell modelliert werden kann, um sie an den Körperteil des Massage-Empfängers anatomisch anzupassen.

2. Vibrationsmassagegerat nach Anspruch 1, **dadurch gekennzeichnet, dass** genannte vibrationsplatte (11) eine kleine Platte (15) umfasst, die mit Befestigungsmitteln (16) versehen ist und an genanntem Gerüst (14) derart angebracht ist, dass der Motorvibrator (12) mit genannter Vibrationsplatte (11) fest verankert ist.

3. Vibrationsmassagegerät nach Anspruch 1 und/oder 2, **dadurch gekennzeichnet, dass** genannte Armatur (14) aus einer biegsamen Metall-Lochplatte mit Löchern unterschiedlichen Durchmessers besteht, die in genannte Vibrationsplatte eingelagert ist.

4. Vibrationsmassagegerät nach Anspruch 2 und/oder 3, **dadurch gekennzeichnet, dass** genannte kleine Platte (15) ein steifes Metallblech ist, das mit Befestigungsmitteln (16) versehen ist und in genannte Vibrationsplatte (11) eingelagert ist, während die genannten Befestigungsmittel aus Schrauben (16) bestehen, die teilweise über die obere Seite der genannten Platte (11) hinausragen, mit den jeweiligen freien Enden durch entsprechende am Untergestell (12.2) des Motorvibrators (12) vorgesehenen Durchgangslöcher (12.1) verlaufen und mittels entsprechender Schraubenmuttern (16.1) den Motorvibrator (12) an die genannte Vibrationsplatte (11) fest verankern.

## Revendications

1. Appareil de massage vibratoire pour la réalisation de massages à des fins thérapeutiques et esthétiques, de réhabilitation, de préparation sportive, comprenant :
- au moins une unité de massage vibratoire (10), incluant une plaque de massage vibratoire (11) et un vibrateur motorisé (12) connecté de manière stable par rapport à ladite plaque (11), et
- une unité de contrôle (20), dotée de dispositifs à circuits électriques pour la connexion de ladite au moins une unité de massage vibratoire (10) par rapport aux moyens d'alimentation électrique et contrôlée à distance par un dispositif de télécommande (17),
de sorte que, durant le traitement, l'appareil ne nécessite aucune intervention de l'opérateur et qu'il est contrôlé par ledit dispositif à télécommande (17), par la personne qui reçoit le massage,
**caractérisé par le fait que** ladite plaque de massage vibratoire (11) est en in silicone et comprend une armature (14) en grille métallique ou une plaque métallique flexible et percée noyée dans ladite plaque de massage vibratoire (11), de sorte qu'elle peut être modelée manuellement pour l'adapter anatomiquement à la partie du corps de la personne à masser.

2. Appareil de massage vibratoire selon la revendication 1, **caractérisé par le fait que** ladite plaque de massage vibratoire (11) comprend une plaquette (15), portant des dispositifs de fixation (16) et appliquée contre ladite armature (14), de manière à assurer solidement le vibrateur motorisé (12) à ladite plaque de massage vibratoire (11).

3. Appareil de massage vibratoire selon la revendication 1 et/ou 2, **caractérisé par le fait que** ladite armature (14) est constituée par une plaque métallique flexible et percée, avec des trous de différents diamètres, laquelle est noyée dans ladite plaque de massage vibratoire.

4. Appareil de massage vibratoire selon la revendication 2 et/ou 3, **caractérisé par le fait que** ladite plaquette (15) est une petite tôle métallique rigide, qui porte des dispositifs de fixation (16) et qui est noyée dans ladite plaque de massage vibratoire (11), tandis que lesdits dispositifs de fixation se composent de vis (16) qui se prolongent en partie au-delà de la face supérieure de ladite plaque (11), traversent avec leurs extrémités libres respectives les trous passants correspondants (12.1), prévus dans l'embase (12.2) du vibrateur motorisé (12), et qui assurent solidement le vibrateur motorisé (12) sur ladite plaque de massage vibratoire (11) grâce à des écrous correspondants (16.1).
